# EUROPEAN PATENT APPLICATION

(11) **EP 2 826 506 A1**
(43) Date of publication of application: **21.01.2015**
(21) Application number: 13176831.9
(22) Date of filing: 17.07.2013
(51) Int. Cl.: A61M 5/145, A61M 5/168

(54) **A pressure-sensing device**

(71) Applicant: Waterford Institute Of Technology, Waterford (IE)
(72) Inventor: The designation of the inventor has not yet been filed
(74) Representative: Lucey, Michael

(57) **Abstract**

A pressure-sensing device for use with a syringe, the device comprising a housing configured to engage with an outlet of the syringe by an engagement means, a tactile sensor in fluid communication with the syringe, and a display means adapted to be connected to the tactile sensor, wherein the display means responds to pressure acting on the tactile sensor.

## Description

### Field of the Invention

The invention relates to a pressure-sensing device. In particular, the invention relates to a pressure-sensing device for use in angioplasty applications.

### Background to the Invention

Coronary Heart Disease (CHD), also known as the Coronary Artery Disease (CAD), is presently the leading cause of death worldwide. The share of Coronary Heart Disease in terms of the total number of deaths is expected to reach 15% by the year 2030. As a result, demand for percutaneous procedures, such as percutaneous transluminal coronary angioplasty (PTCA) used in treating the condition, is expected to increase in the ensuing years, as incidence and prevalence of the condition rises. PTCA is a globally widespread procedure for treating heart disease and is experiencing rapid yearly growth rates. PTCA involves the inflation of a balloon inside an obstructed vessel to deploy a stent. Coronary angioplasty and Coronary Artery Bypass Graft (CABG) represent the two major forms of revascularization procedures, which received much attention over the recent decades, and have revolutionized the heart disease treatment worldwide.

Presently, about 3.0 million angioplasty procedures are performed each year worldwide, with the US alone accounting for almost a million of these procedures. PTCA and coronary stenting products are expected to increase at a compound annual rate of 6.6%. Within the global medical community there is a growing demand for cost effective medical devices.

Currently, a manually operated pump attached to a vessel, and a pressure pump monitor, are the most widely used techniques for delivering pressurised solution to the catheter. A feedback sensor is used to monitor pressure within the balloon. Images of the patient's vascular system are displayed on a fluoroscope with the aid of a radiopaque liquid, allowing the surgeon to operate and react in real-time. A number of devices already exist to fulfil the function of balloon inflation/deflation. Typically, these devices require two handed operation with one hand holding the shaft of the device while the other operates a rotating mechanism to increase or decrease the pressure. This reliance on a screw-thread mechanism to apply mechanical force, which in turn is translated into an increase/decrease in pressure, is inherently flawed. It is inconvenient for the surgeon to use, can lead to distractions, and is difficult to manipulate in case of an emergency (*i.e.* in the event that immediate depressurization is required). Furthermore, existing techniques are entirely devoid of tactile feedback and the surgeon is reliant on a peripherally located pressure gauge for visual feedback on operational pressures. This is a further distraction as it requires the surgeon to momentarily take their attention away from the operation at hand.

Examples of such devices can be found in patent documents EP 0396353 B1, where a pressure transducer is incorporated into existing inflation device components and electronically coupled to an illuminated digital display, and US 6234996 where a low volume syringe plunger is operated by a rotary dial containing some form of indicia on the outer device casing.

There is therefore a need to provide alternative inflation device technology. It is an object of the present invention to overcome at least some of the above-mentioned problems.

### Summary of the Invention

According to the present invention there is provided, as set out in the appended claims, a pressure-sensing luer device for use with a pressure-sensing device for use with a syringe, the device comprising:
a body configured to engage with an outlet of a valve by an engagement means;
a valve configured to engage with a syringe; and
a display means adapted to be in fluid communication with the valve, wherein the display means responds positively or negatively to a pressure value acting from the valve.

In one embodiment, the display means comprises a traffic light status indicator.

In one embodiment, the device further comprises a tactile sensor.

In one embodiment, the tactile sensor comprises a spring and plunger configuration.

In one embodiment, the plunger is engaged with the spring and adapted to respond to an increase or decrease in pressure.

In one embodiment, the device further comprises a tensioning means configured to adjust and lock the tension of the tactile sensor at a selected pressure value.

In one embodiment, the tactile sensor comprises a pressure sensor.

In one embodiment, the pressure sensor is powered by a power source.

In one embodiment, the pressure value is in the pressure range of -0.1 MPa to 3 MPa.

In one embodiment, the tactile sensor and display means are integrated into the body of the device.

In one embodiment, the engagement means is a luer locking mechanism.

In one embodiment, the device is retrofittable to any syringe.

In one embodiment, the pressure-sensing device is a single-use device pre-sterilised before connecting to the syringe.

In one embodiment, the body is constructed from a durable yet rigid material, for example polypropylene, polycarbonate, polyisoprene polyethylene (PE), polyethylene terephthalate copolymer (PETG), amorphous polyethylene terephthalate (APET).

In one embodiment, the spring is made from, for example, spring steel (also known as music wire).

In one embodiment a check valve (non-return valve) is in place of the 4-way valve, where a pinch valve is in parallel with the check valve to relieve the pressure stored by the check valve. This pinch valve is a one finger operation. In another embodiment, a ratchet mechanism is used (similar to a silicone gun), where inflation is incremental and operated using one hand alone, with a pressure release valve located in proximity to the ratchet handle.

In the specification, the term "tactile sensor" should be understood to mean a device which receives and responds to a signal or stimulus having to do with force.

In the specification, the term "traffic light status indicator" should be understood to mean the use of an indicator resembling a traffic light system which is generally visible when a person is using the device. Green typically indicates acceptable pressure or more pressure is permitted; amber indicates that pressure is building to a desired level or attention to the pressure being applied by user on the syringe is otherwise warranted; and red indicates that pressure is approaching the desired level or the desired pressure level has been reached and no more pressure should be applied. The letters R, A and G can also be used in addition to swatches of colour, so that the system can be used by colour blind users. Other visual indicators such as numbers, letters, codes or colours could also be used in place of the "traffic light status indicator".

In the specification, the term "luer locking mechanism" should be understood by those skilled in the art. The term is applied to luer lock fittings securely joined by means of a tabbed hub on the female fitting which screws into threads in a sleeve on the male fitting. This forms a secure and locked fitting, preventing the joined components from coming apart, even under pressure from a fluid being forced between the two components.

In the specification, the term "fluid" should be understood to mean a substance that is capable of flowing and that changes its shape at a steady rate when acted upon by a force, such as, for example, a gas, a liquid, or an oil.

In the specification, the term "tensioning means" should be understood as the application of a linear adjustment to a spring with the aim of changing a set-point, whereby the tension can be adjusted to a desired pressure and where the pressure and sensor can be maintained at that tension during use.

In the specification, the term "selected pressure value" should be understood to mean a pressure value in the pressure range of -0.1 MPa to 3 MPa.

In the specification, the term "retrofittable" should be understood to mean that the device of the present invention can be attached to any syringe currently being used in the medical or other fields.

One of the advantages of the present invention is that (i) the device permits one-handed operation in a manageable and easy fashion, (ii) the device provides tactile feedback, (iii) the pressure display functions of the device occur directly in the user's field of vision, (iv) the device is comprised of commodity technologies, therefore low cost, and (v) the device is retrofittable (to standard syringes) as it contains a standardized, interchangeable, connection port.

The disclosed invention offers an effective, low cost alternative to existing inflation devices or fluid flow monitoring devices and in doing so provides a more efficiently designed, cost effective solution, to the medical/fluid flow dynamics community.

### Brief Description of the Drawings

The invention will be more clearly understood from the following description of an embodiment thereof, given by way of example only, with reference to the accompanying drawings, in which:-
**Figure 1** illustrates a pressure sensing device of the claimed invention connected to a standard 5ml syringe through a standard 3-way valve. The small syringe will enable the physician to develop high pressures with low cost standard syringes. The three way valve can be used to "lock" / maintain the pressure within the system. By simply opening the valve the clinician can increase or reduce the pressure within the system by applying or removing force from the plunger.
**Figure 2** illustrates a cut-away section of one embodiment of the pressure sensing device of the claimed invention where the indicator is visible on the piston plunger of the device;
**Figure 3** illustrates a cut-away section of one embodiment of the pressure sensing device of the claimed invention where the indicator is visible in the body of the device;
**Figure 4** illustrates an exploded view of the pressure sensing device of the claimed invention;
**Figure 5** illustrates a high pressure 4-way valve for use with the pressure sensing device of the claimed invention. At the 9 o clock position this will allow the inflation syringe to fill both an inflation catheter and pressure sensing device. By turning the valve to the 6 o clock position it will act as a pressure lock, maintaining the pressure within the system and allowing the free exchange of syringes from the 4-way stopcock;
**Figure 6** illustrates that pressures in excess of 30 Bar (3 MPa) can be generated using a 1ml Polycarbonate Syringe;
**Figure 7A, 7B** and **7C** illustrate the sensing device being tested through a range of pressures from (A) 0 bar (0 MPa) to (C) approximately 34 bar (approximately 3.4 MPa);

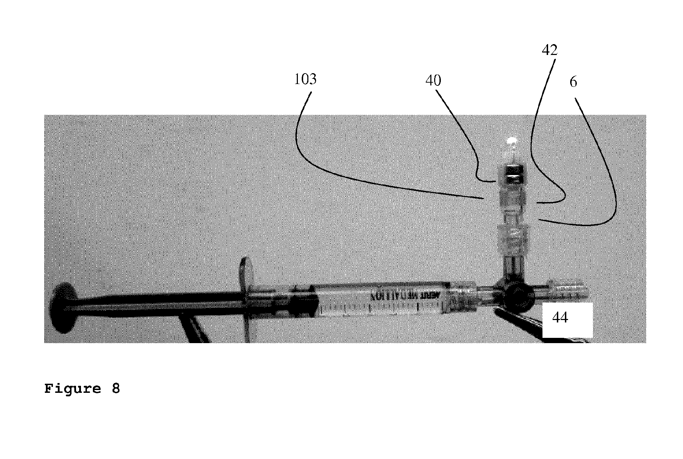

**Figure 8** illustrates a sensing device of the claimed invention with an electronic indicator.

### Detailed Description of the Drawings

The present invention provides a retrofittable pressure sensing device for angioplasty applications, as used in the inflation of balloon catheters during intravenous surgical procedures such as stent placement and deployment. Specifically, the device is designed for use in conjunction with standard medical syringes. The device in question consists of a spring and plunger configuration, and a tensioning means (for calibrating the spring-plunger arrangement), all of which are housed in the main body of the device. The main body itself contains an affixed connection port at the distal end, for interfacing to a standard syringe. The spring-plunger mechanism is responsible for pressure sensing and display functions with the latter communicated based on the globally recognised 'traffic light' warning system. The pressure sensing and display apparatus may be integrated into the main body of the device or in additional embodiments may exist as a separate module. The syringe is used to inflate the angioplasty balloon once it has reached its correct location in the vessel using a saline solution. A pressurised saline solution is preferred over air due to the possibility of air bubbles escaping into the bloodstream in the event of balloon failure. The device can be used in direct contact with the fluid or connected through a transducer medium. The device operates in the pressure range of -0.1 MPa to 3 MPa and is capable of delivering 10-20ml of saline solution/radiopaque dye.

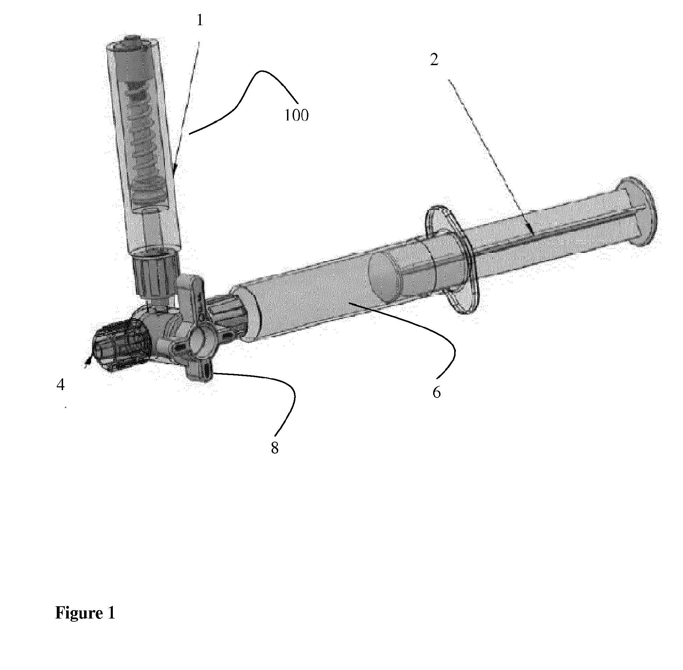

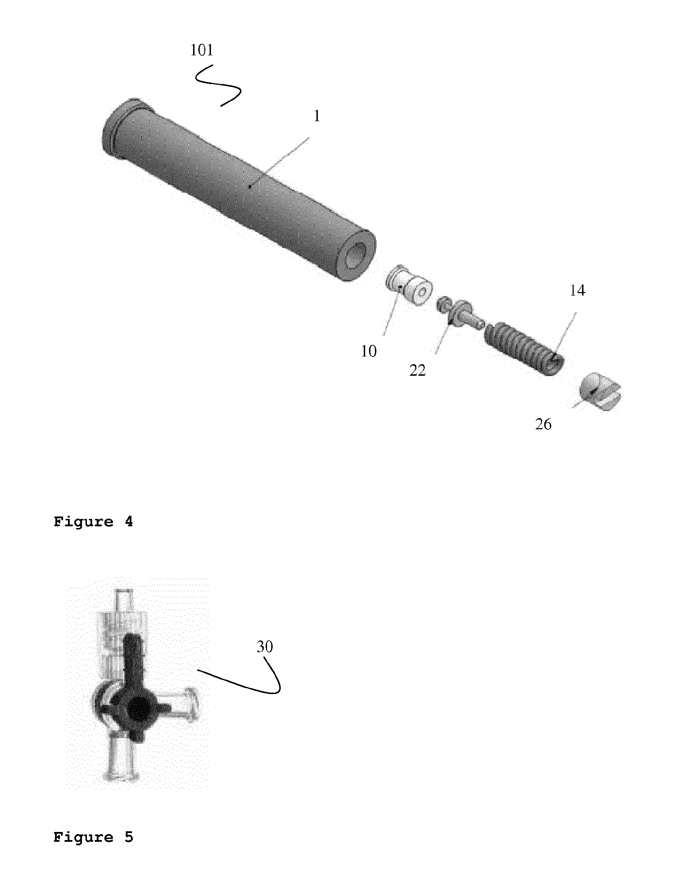

Referring now to the figures, where **Figure 1** illustrates a general embodiment of a pressure-sensing device of the present invention. Specifically, **Figure 1** illustrates a perspective view of the pressure sensing device of the present invention attached to a standard 5ml syringe, and is generally referred to by reference numeral 100. The pressure sensing device 100 comprises a pressure indicator 1 coupled to a 3-way valve 8 (or a 4-way valve 30, see Figure 5). The 3-way valve 8 has a luer lock connection port 4 for connecting to a balloon or the like. The standard syringe 2 comprises a plunger 5 and body 6 adapted to accept the plunger 5 internally. The syringe 2 connects to the 3-way valve 8 via the male/female luer lock system.

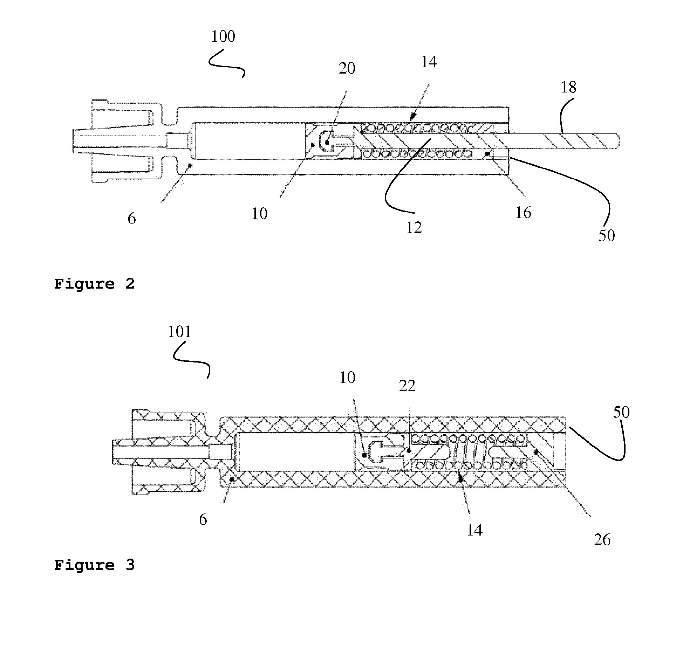

As illustrated in **Figure 2****,** there is presented a cut-away section of the device 100. The device 100 comprises a main body 6, a plunger 12, a piston plunger seal 10, a torsion means 16 and a spring 14. The plunger 12 is configured at its proximal end 20 to engage with the seal 10 and is further configured at its distal end 18 to act as a traffic light status indicator 32 for the device 100. The seal 10 acts to prevent fluid from escaping the device 100 when in use or at rest. The plunger 12 is further encompassed by the spring 14. The spring 14 is biased to be in a relaxed state and rests between the seal 10 and torsion means 16. The torsion means 16 is at the distal end 50 of the device 100. The torsion means 16 allows the user to calibrate the device 100 to accept a specific pressure by putting force on or allowing force off the spring 14.

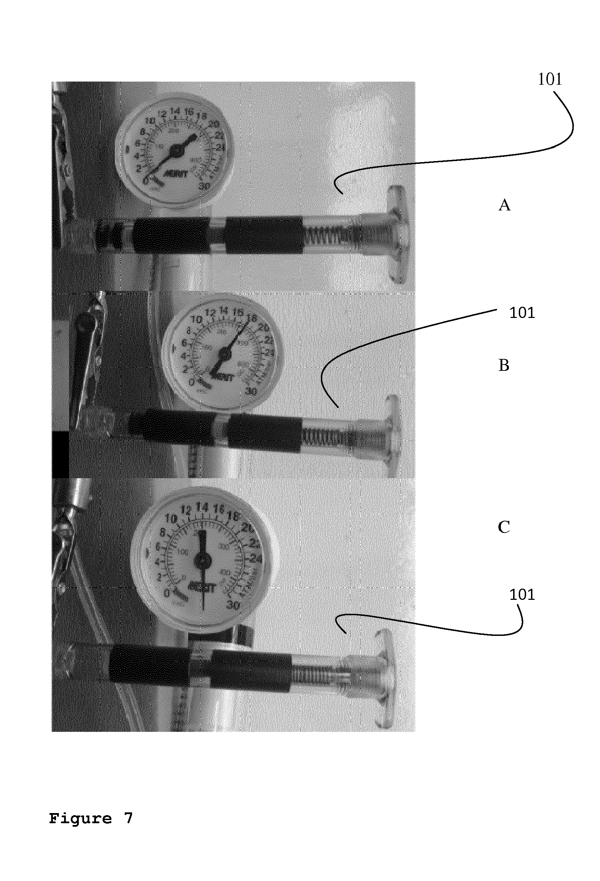

**Figure 3** illustrates another embodiment of the device 101 of the present invention. The device 101 depicted in Figure 3 comprises a main body 6, a plunger 22, a piston plunger seal 10, a torsion means 26 and a spring 14. The plunger 22 is configured to engage with the seal 10 and the spring 14. The torsion means 26 is configured to engage with the spring 14 opposite the plunger 22. The traffic light status indicator for the device 101 is configured to engage with and between the plunger 22 and torsion means 26. The plunger 22 and torsion means 26 are encompassed by the spring 14. The spring 14 is biased to be in a relaxed state. The torsion means 26 is at the distal end 50 of the device 101. The torsion means 26 allows the user to calibrate the device 101 to accept a specific pressure by putting force on or allowing force off the spring 14. Although not shown here, the traffic light status indicator 32 engages with the outer circumference of the spring 14 and is visible in a window 34 on the body 6 of the device 101 (see Figure 7).

In order to calibrate the device 100, 101, the method consists of a standard inflation balloon (or any similar device) (Merit Medicals Basix Compak) with a calibrated pressure gauge. This is connected to the device 100, 101 through the 3-way valve 8, the other port on the 3-way valve can be connected to a standard 5ml Leur-Lok^{™} syringe 2 for high pressure demonstration purposes.

This device 100, 101 is calibrated using the following steps:
- The device 100, 101 is filled with water.
- All air is purged from the device 100, 101.
- The standard inflation balloon is brought to a predetermined pressure (*e.g*. 10bar (1 MPa)).
- The tensioning means 16, 26 is adjusted as required to indicate the correct level.
- The pressure is adjusted through a full range of pressures on the inflation balloon.

**Figure 4** illustrates an exploded view of the device 101 of the claimed invention where the traffic light indicator is missing.

**Figure 5** illustrates a high pressure 4-way valve 30 for use with the device 100, 101 of the present invention. At the 9 o'clock position, the valve 30 will allow the syringe 2 to fill both an inflation catheter and device 100, 101. By turning the valve 30 to the 6 o'clock position, the valve 30 will act as a pressure lock, maintaining the pressure within the system and allowing the free exchange of syringes from the 4-way valve 30.

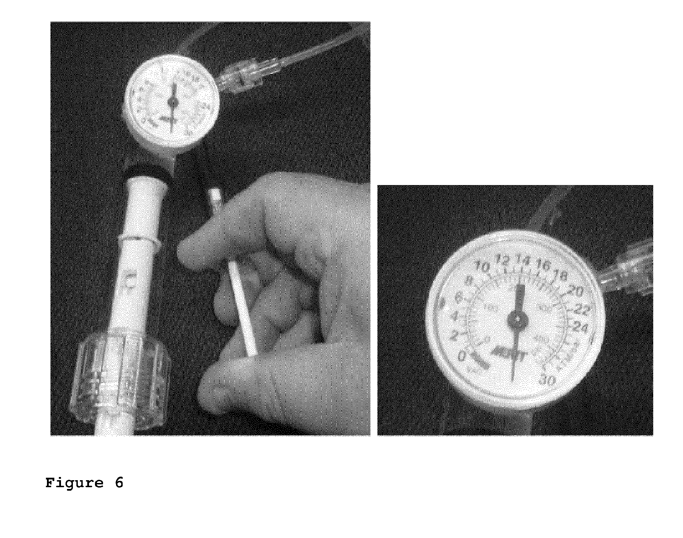

In use, as illustrated in **Figures 6** **and** **7****,** the syringe 2 used can generate the necessary pressures, as shown in the pressure gauge. The traffic light status indicator 32 used in the device 101 is depicted in Figure 7A shows that when the pressure is at 0 bar (0 MPa), the spring 14 is in a relaxed state and the indicator 32 visible in the window 34 is indicating a colour green to prompt the user to apply more pressure. When pressure of approximately 16 bar (1.6 MPa) is applied (Figure 7B), the amplitude in the spring 14 is noticeably shortening and the colour of the indicator 32 changes to a warning colour prompting the user to begin to stop applying pressure. The amplitude of the spring 14 has shortened even further in Figure 7C when pressure in excess of 30 bar (3 MPa) is applied. A warning colour, for example red, is visible on the indicator 32 through window 34.

In another embodiment of the invention, the device 103 can comprise a main body 6, a pressure sensor 40, a battery source 44 and a traffic light status indicator 42. The indicator 42 can be in the form of a variable colour light-emitting diode (LED). The components are built inside the body 6 with a luer lock connection. The device 103 is configured to connect to the 4-way valve 30 and is activated when pressure reaches set levels.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A pressure-sensing device for use with a syringe, the device comprising:
a body configured to engage with an outlet of a valve by an engagement means;
a valve configured to engage with a syringe; and
a display means adapted to be in fluid communication with the valve, wherein the display means responds positively or negatively to a pressure value acting from the valve.

2. A pressure-sensing device according to Claim 1, wherein the display means comprises a traffic light status indicator.

3. A pressure-sensing device according to Claim 1 or Claim 2, wherein the device further comprises a tactile sensor.

4. A pressure-sensing device according to Claim 3, wherein the tactile sensor comprises a spring and plunger configuration.

5. A pressure-sensing device according to Claim 4, wherein the plunger is engaged with the spring and adapted to respond to an increase or decrease in pressure.

6. A pressure-sensing device according to any preceding Claim, wherein the device further comprises a tensioning means configured to adjust and lock the tension of the tactile sensor at a selected pressure value.

7. A pressure-sensing device according to Claim 3, wherein the tactile sensor comprises a pressure sensor.

8. A pressure-sensing device according to Claim 7, wherein the pressure sensor is powered by a power source.

9. A pressure-sensing device according to any one of the preceding claims, wherein the pressure value is in the pressure range of -0.1 MPa to 3 MPa.

10. A pressure-sensing device according to any preceding claim, wherein the tactile sensor and display means are integrated into the body of the device.

11. A pressure-sensing device according to any preceding claim, wherein the engagement means is a luer locking mechanism.

12. A pressure-sensing device according to any preceding claims, wherein the device is retrofittable to any syringe.

13. A pressure-sensing device according to any preceding claim, wherein the pressure-sensing device is a single-use device pre-sterilised before connecting to the syringe.

14. A pressure-sensing device according to any preceding means, wherein the body is constructed from a durable yet rigid material, for example polypropylene, polycarbonate, polyisoprene polyethylene (PE), polyethylene terephthalate copolymer (PETG), amorphous polyethylene terephthalate (APET).
